# EUROPÄISCHE PATENTSCHRIFT

(11) **EP 0 805 631 B1**
(45) Veröffentlichungstag und Bekanntmachung des Hinweises auf die Patenterteilung: **06.05.1999**
(21) Anmeldenummer: 96901272.3
(22) Anmeldetag: 16.01.1996
(51) Int. Cl.: A23J 3/34, A61K 7/48

(54) **VERFAHREN ZUR HERSTELLUNG VON WEIZENPROTEINHYDROLYSATEN**
PROCESS FOR PRODUCING WHEAT PROTEIN HYDROLYSATES
PROCEDE DE PRODUCTION D'HYDROLYSATS DE PROTEINES DU BLE

(30) Priorität: 25.01.1995 DE 19502168
(43) Veröffentlichungstag der Anmeldung: 12.11.1997
(73) Patentinhaber: Henkel Kommanditgesellschaft auf Aktien, 40191 Düsseldorf (DE)
(72) Erfinder: VON KRIES, Edith, D-89257 Illertissen (DE); HEILEMANN, Andrea, D-89079 Ulm (DE); SANDER, Andreas, D-89257 Illertissen (DE)
(86) Internationale Anmeldenummer: EP9600147
(87) Internationale Veröffentlichungsnummer: WO9622699

(56) Entgegenhaltungen:
- EP-A- 0 298 419
- EP-A- 0 495 391
- EP-A- 0 578 572
- DE-A- 4 116 744
- DE-C- 4 410 000
- FR-A- 2 688 229
- US-A- 4 138 500
- PATENT ABSTRACTS OF JAPAN vol. 013, no. 003 (C-557), 6.Januar 1989 & JP,A,63 216438 (JAPANESE RES & DEV ASSOC BIO REACTOR SYST FOOD IND), 8.September 1988,
- PATENT ABSTRACTS OF JAPAN vol. 014, no. 086 (C-0690), 19.Februar 1990 & JP,A,01 300854 (JAPANESE RES & DEV ASSOC BIO REACTOR SYST FOOD IND), 5.Dezember 1989,

## Beschreibung

### Gebiet der Erfindung

Die Erfindung betrifft ein Verfahren zur Herstellung von Weizenproteinhydrolysaten, bei dem man die mehrstufige Hydrolyse in Gegenwart ausgewählter Enzyme durchführt sowie die Verwendung der Hydrolysate zur Herstellung hellfarbiger, lagerstabiler Derivate.

### Stand der Technik

Abbauprodukte von Polypeptiden, sogenannte Proteinhydrolysate, sind seit langem bekannt. Obschon sie wegen des Fehlens einer lipophilen Gruppe keine Detergenseigenschaften besitzen, werden sie wegen ihrer dispergieren Eigenschaften und ihrer Fähigkeit, die dermatologische Verträglichkeit anionischer Tenside durch Wechselwirkung mit den Eiweißmolekülen der Haut günstig zu beeinflussen, in einer Vielzahl von oberflächenaktiven Mitteln eingesetzt. Übersichtsartikel hierzu finden sich beispielsweise von A.Domsch et al. in **Ärztl**. **Kosmetol**. **13**, **524 (1983)**, G.Schuster et al. in **Cos-met**.**Toil**., **99**, **12 (1984)** und H.Lindner in **Parfüm**.**Kosmet**., **66**, **85 (1985)**.

Üblicherweise werden Proteinhydrolysate auf Basis von tierischem Kollagen gewonnen. In den letzten Jahren hat sich jedoch ein Trend nach pflanzlichen Produkten, beispielsweise auf Basis von Weizengluten oder Sojaprotein durchgesetzt.

Aus der französischen Offenlegungsschrift **FR 2542013** (ABC) ist beispielsweise die Hydrolyse pflanzlicher Proteine mittels besonderer Milchsäurebakterien in Gegenwart von Kohlenwasserstoffen bekannt. In der **US 4757007** (Nisshin) wird die partielle Hydrolyse von Sojaproteinen mit Proteasen in Fraktionen unterschiedlicher Löslichkeit in Trichloressigsäure, Trennung der Fraktionen bei einem pH-Wert von 7, Abtrennung nichthydrolysierter Anteile und Reinigung der Produkte durch Ultrafiltration beschrieben. Gegenstand der europäischen Patentanmeldung **EP-A 0187048** (Novo) ist der enzymatische Abbau von Sojaproteinen durch Behandlung mit speziellen Proteasen. Aus der **EP-A 0298419** (Katayama) ist die Herstellung von Proteinhydrolysaten mit einem durchschnittlichen Molekulargewicht von 500 bis 90.000 durch schrittweisen alkalischen, sauren und/oder enzymatischen Abbau von Weizen- oder Sojaproteinen bekannt. In der **EP-A 0363771** (Nestlé) wird schließlich über ein Verfahren zur Herstellung von Proteinhydrolysaten berichtet, bei dem man pflanzliche Proteine mit Salzsäure hydrolysiert, nichthydrolysierte Bestandteile abtrennt, zur Zerstörung unerwünschter chlorierter Verbindungen alkalisch stellt und die resultierenden Produkte anschließend ansäuert.

Den Verfahren des Stands der Technik ist jedoch gemein, daß sie angewendet auf den pflanzlichen Rohstoff Weizen Produkte liefern, die nach chemischer Derivatisierung, beispielsweise nach Kondensation mit Fettsäurechloriden, verfärbten und nicht ausreichend lagerstabil sind. Ein besonderes Problem besteht beispielsweise darin, daß Fettsäurekondensate von Weizenproteinhydroylsaten des Stands der Technik in wäßriger Lösung eine unerwünschte Tendenz zur Austrübung zeigen.

Die Aufgabe der Erfindung hat somit darin bestanden, für das Problem der unzureichenden Lagerstabilität von Derivaten auf Basis von Weizenproteinhydrolysaten eine Abhilfe zu schaffen.

### Beschreibung der Erfindung

Gegenstand der Erfindung ist ein Verfahren zur Herstellung von Weizenproteinhydrolysaten, bei dem man proteinhaltige Ausgangsstoffe
(a) zunächst bei einem pH-Wert im Bereich von 2 bis 5 mit Proteinasen und
(b) anschließend bei einem pH-Wert im Bereich von 8 bis 10 mit Proteinasen behandelt, sowie
(c) abschließend bei einem pH-Wert im Bereich von 6 bis 7 mit Peptidasen hydrolysiert.

Nach umfangreichen Untersuchungen der Anmelderin hat sich gezeigt, daß die unzureichende Lagerstabilität der Derivate auf eine unvorteilhafte Molgewichtsverteilung der Vorstufe - also der Weizenproteinhydrolysate - zurückzuführen ist. Demzufolge mußte die Lösung der gestellten Aufgabe auf der Stufe der Weizenproteinhydrolysate greifen. Überraschenderweise wurde gefunden, daß ein enzymatischer Abbau unter besonderer Auswahl der eingesetzten Enzyme zu Hydrolysaten führt, die ihrerseits lagerstabile Derivate, insbesondere in wäßriger Lösung trübungsfreie Weizenproteinfettsäurekondensate ergeben.

In einer bevorzugten Ausführungsform des erfindungsgemäßen Verfahrens werden als proteinhaltige Ausgangsstoffe, Weizengluten sowie entsprechende chemisch bzw. enzymatisch modifizierte Derivate hydrolysiert.

Unter proteinhaltigen Ausgangsstoffen sind Proteinisolate zu verstehen, die beispielsweise durch Extraktion von Weizenmehl nach bekannten Verfahren des Stands der Technik erhalten werden und einen Proteingehalt im Bereich von 70 bis 90 Gew.-% aufweisen können.

### Proteinasen und Peptidasen

Proteinasen und Peptidasen zählen zur Gruppe der Proteasen, also Enzymen, welche die hydrolytische Spaltung der Peptidbindung katalysieren und daher systematisch gesehen zu den Hydrolasen gehören. Proteinasen, die auch als Endoproteasen oder Endopeptidasen bezeichnet werden, spalten Peptidbindungen im Inneren des Proteins. Sie sind von den (Exo-)Peptidasen zu unterscheiden, die einen Abbau der terminalen Peptidbindung von der endständigen Amino- bzw. Carboxylgruppe bewirken.

Typische Beispiele für im Sinne des erfindungsgemäßen Verfahrens geeignete **Proteinasen** sind die im Handel erhältlichen Serin-Proteinasen (EC 3.4.21), Cystein- bzw. Thiol-Proteinasen (EC 3.4.22), saure Proteinasen vom Typ der Aspartat- bzw. Carboxyproteinasen (EC 3.4.23) sowie untergeordnet auch Metall-Proteinasen (3.4.24). Beispiele für für geeignete Serin-Proteinasen sind Chymotrypsin, Elastase, Kallikrein, Plasmin, Trypsin, Thrombin und Subtilisin.

Zu den geeigneten **Peptidasen** zählen beispielsweise die α-Ami- noacylpeptid-Hydrolasen oder Aminopeptidasen (EC 3.4.11), die am Ende des Polypeptids einzelne Aminosäuren ablösen, die Di- peptid-Hydrolasen bzw. Dipeptidasen (EC 3.4.13), die Dipepti- de zu Aminosäuren hydrolysieren, die Dipeptidylpeptid-Hydro- lasen bzw. Dipeptidylpeptidasen (EC 3.4.14), die Amino-stän- dige Dipeptide eines Polypeptids freisetzen, Peptidyldipeptid-Hydrolasen bzw. Dipeptidylcarboxypeptidasen (EC 3.4.15), die einzelne Aminosäuren des Carboxy-Terminus abtrennen, Carboxypeptidasen (EC 3.4.16 - 3.4.18) und Omega-Peptidasen (EC 3.4.19), die modifizierte Aminosäuren von beiden Ende des Polypeptids abspalten.

Die Menge der eingesetzten Proteinasen bzw. Peptidasen ist an sich nicht kritisch, sollte jedoch jeweils im Bereich von 0,1 bis 5, vorzugsweise 0,5 bis 2 Gew.-% - bezogen auf die Ausgangsstoffe - liegen.

### Adsorbentien

Zur Entfernung von Spuren an unerwünschten Farbverursachern hat es sich als vorteilhaft erwiesen, die proteinhaltigen Ausgangsstoffe zusammen mit geeigneten Adsorbentien in die Hydrolyse einzusetzen. Als Adsorbentien kommen beispielsweise Kieselgele, Aluminiumoxide und vorzugsweise Aktivkohlen in Betracht, die in Mengen von 0,1 bis 15, vorzugsweise 1 bis 5 Gew.-% - bezogen auf den Stickstoffgehalt der proteinhaltigen Ausgangsstoffe - eingesetzt werden können.

### Durchführung des Hydrolyseverfahren

Zur Durchführung der enzymatischen Hydrolyse wird eine wäßrige Suspension des proteinhaltigen Ausgangsstoffs gegebenenfalls zusammen mit den Adsorbentien wie oben beschrieben über einen Zeitraum von 1 bis 24 h im Temperatur- und pH-Wertoptimum der eingesetzten Proteinasen und Peptidasen, beispielsweise bei 40 bis 70°C abgebaut. Besonders vorteilhaft ist es, die Hydrolyse unterhalb der Verkleisterungstemperatur der im Protein noch einhaltenen Kohlenhydrate durchzuführen.

Wird der Aufschluß in Gegenwart von Calciumoxid bzw. Calciumhydroxid als Base durchgeführt, bilden sich lösliche Calciumpeptide, die vom ungelösten Calciumoxid oder Calciumhydroxid durch Filtration abgetrennt werden müssen. Werden die Alkalipeptide gewünscht, empfiehlt es sich, die Calciumpeptide mit Soda- oder Pottaschelösung zu behandeln und das schwerlösliche Calciumcarbonat anschließend abzutrennen. Es ist ebenfalls möglich, das Calcium in Form von Calciumsulfat oder Calciumoxalat zu fällen. Die Abtrennung der schwerlöslichen Salze erfolgt vorzugsweise in Gegenwart von Filterhilfsmitteln mit den üblichen Trennverfahren für Fest/Flüssig-Trennungen wie Filtration, Separation und dergleichen.

Es werden wäßrige Weizenproteinhydrolysatlösungen erhalten, die nach Bedarf beispielsweise unter Einsatz von Fallstromverdampfern aufkonzentriert werden können. Die nach dem erfindungsgemäßen Verfahren erhältlichen Hydrolysate weisen ein mittleres Molekulargewicht im Bereich von 100 bis 30.000, vorzugsweise 100 bis 10.000 und insbesondere 2000 bis 5000 auf sowie einen Feststoffgehalt von etwa 5 bis 50 Gew.-%.

### Gewerbliche Anwendbarkeit

Die nach dem erfindungsgemäßen Verfahren erhältlichen pflanzlichen Weizenproteinhydrolysate zeichnen sich durch eine besonders vorteilhafte Farbqualität aus und liefern nach Derivatisierung Stoffe, die in wäßriger Lösung eine besonders hohe Lagerstabilität zeigen. Insbesondere Fettsäurekondensate auf Basis der nach dem erfindungsgemäßen Verfahren erhältlichen Weizenproteinhydrolysaten lassen sich zu klaren, lagerstabilen wäßrigen Lösungen verarbeiten.

Ein weiterer Gegenstand der Erfindung betrifft daher die Verwendung der nach dem erfindungsgemäßen Verfahren erhältlichen Weizenproteinhydrolysate zur Herstellung von hellfarbigen, lagerstabilen Folgeprodukten wie beispielsweise N-acylierten, N-alkylierten, veresterten sowie N-acylierten bzw. N-alkylierten und zudem veresterten Derivaten.

Vorzugsweise werden die nach dem erfindungsgemäßen Verfahren erhältlichen Weizenproteinhydrolysate in an sich bekannter Weise mit Fettsäuren bzw. Fettsäurechloriden mit 6 bis 22, insbesondere 12 bis 18 Kohlenstoffatomen kondensiert. Besonders bevorzugt ist die Verwendung der Weizenproteinhydrolysate zur Herstellung von Laurinsäure- bzw. Kokosfettsäurekondensaten.

Die folgenden Beispiele sollen den Gegenstand der Erfindung näher erläutern, ohne ihn darauf einzuschränken.

### Beispiele

### Beispiel 1:

In einem 5-m³-Rührkessel wurden 3500 l Warmwasser (Tₘₐₓ = 50°C) vorgelegt und mit 1,3 kg Natriumsulfit und 14 kg Aktivkohle versetzt. Bei 48 bis 50°C wurden bei maximaler Rührerdrehzahl 650 kg Weizenproteinisolat zugesetzt und zu einer Suspension verrührt. Danach wurde der pH-Wert der Reaktionsmischung durch Zugabe von Salzsäure auf pH = 3,0 eingestellt.

Anschließend wurden 5 kg Proteinase mit einem pH-Optimum im sauren Bereich zugegeben. Bei der Hydrolyse und der sich anschließenden ersten Filtration wurde die Temperatur auf maximal 50°C begrenzt und die Sulfitkonzentration oberhalb vom 10 ppm gehalten. Durch Zugabe von Salzsäure wurde der pH-Wert zunächst bei 3,0 gehalten und nach 2 h durch Zugabe von Calciumhydroxid auf 8,5 eingestellt. Gleichzeitig erfolgte die Zugabe von weiteren 14 kg Aktivkohle und 5 kg Proteinase mit einem pH-Optimum im alkalischen Bereich. Ohne pH-Wert-Korrektur wurden weitere 2 h bei etwa 50°C gerührt. Nach Abschluß der enzymatischen Hydrolyseschritte wurde der pH-Wert der Mischung durch Zugabe von Calciumhydroxid auf pH = 7,5 eingestellt. Das so hergestellte Hydrolysat wurde nach Zugabe von 70 kg Filterhilfsmittel (Perlite^{(R)} P 50) über eine Filterpresse filtriert.

Anschließend wurden zum Filtrat 20 kg Carbopal^{(R)} GnA gegeben und auf 80°C erhitzt. Diese Temperatur wurde 15 min gehalten. Danach wurde der Ansatz auf 50°C abgekühlt und 30 bei dieser Temperatur gerührt. Nach Zugabe von weiteren 15 kg Filterhilfsmittel wurde noch einmal über eine Filterpresse filtriert. Schließlich wurde das Calcium durch Zugabe von Soda ausgefällt und das Calciumcarbonat über eine Filterpresse abgetrennt. Das Filtrat wurde in einem Fallstromverdampfer bis zu einem Gehalt von 44 % Brix aufkonzentriert. Schließlich wurde das Konzentrat mit Natronlauge auf einen pH-Wert von 10 eingestellt und nach einer Lagerung von 5 Tagen unter Zugabe von 15 kg Filterhilfsmittel über eine Filterpresse filtriert.

### Beispiel 2:

10 kg des gemäß Beispiel 1 hergestellten Weizenproteinhydrolysates wurden in bekannter Weise mit Laurinsäurechlorid acyliert. Das Reaktionsprodukt wurde auf einen Gehalt von 20 Gew.-% Trockensubstanz eingestellt und 3 Wochen bei 20°C und 40°C gelagert. Nach Ablauf der Lagerzeit war das Produkt unter beiden Temperaturbedingungen klar und hatte sich in der Farbe praktisch nicht verändert.

### Vergleichsbeispiel 1:

Ein Weizenproteinhydroylsat des Handels wurde wie in Beispiel 2 beschrieben mit Laurinsäurechlorid acyliert. Eine 20 Gew.-%ige Lösung des resultierenden Weizenproteinfettsäurekondensats trübte bereits nach einer Lagerzeit von 1 Woche aus.

## Patentansprüche

1. Verfahren zur Herstellung von Weizenproteinhydrolysaten, bei dem man proteinhaltige Ausgangsstoffe
(a) zunächst bei einem pH-Wert im Bereich von 2 bis 5 mit Proteinasen und
(b) anschließend bei einem pH-Wert im Bereich von 8 bis 10 mit Proteinasen behandelt, sowie
(c) abschließend bei einem pH-Wert im Bereich von 6 bis 7 mit Peptidasen
hydrolysiert.

2. Verfahren nach Anspruch 1, **dadurch gekennzeichnet**, daß man die dreistufige Hydrolyse in Gegenwart von Aktivkohle durchführt.

3. Verfahren nach den Ansprüchen 1 und 2, **dadurch gekennzeichnet**, daß man die Hydrolyse unterhalb der Verkleisterungstemperatur der im Protein noch enthaltenen Kohlenhydrate durchführt.

4. Verwendung der Weizenproteinhydrolysate erhältlich nach dem Verfahren nach den Ansprüchen 1 bis 3 zur Herstellung von hellfarbigen, lagerstabilen N-acylierten, N-alkylierten, veresterten sowie N-acylierten bzw. N-alkylierten und zudem veresterten Derivaten.

## Claims

1. A process for the production of wheat protein hydrolyzates, in which protein-containing starting materials are hydrolyzed
(a) with proteinases first at a pH value of 2 to 5 and
(b) then at a pH value of 8 to 10 and
(c) finally with peptidases at a pH value of 6 to 7.

2. A process as claimed in claim 1, characterized in that the three-stage hydrolysis is carried out in the presence of active carbon.

3. A process as claimed in claims 1 and 2, characterized in that the hydrolysis is carried out below the gelatinization temperature of the carbohydrates still present in the protein.

4. The use of the wheat protein hydrolyzates obtainable by the process claimed in claims 1 to 3 for the production of light-coloured, storage-stable N-acylated, N-alkylated, esterified and N-acylated or N-alkylated and, in addition, esterified derivatives.

## Revendications

1. Procédé de production d'hydrolysats de protéines de blé dans lequel :
a) on traite en premier lieu à une valeur de pH dans la zone de 2 à 5 avec des protéinases des matières premières contenant des protéines,
b) ensuite à une valeur de pH de 8 à 10 avec des protéinases ainsi que
c) ensuite on hydrolyse à une valeur de pH dans la zone de 6 à 7 avec des peptidases.

2. Procédé selon la revendication 1,
caractérisé en ce qu'
on effectue l'hydrolyse en trois étapes en présence de charbon activé.

3. Procédé selon les revendications 1 et 2,
caractérisé en ce qu'
on effectue l'hydrolyse en dessous de la température de gélification des hydrates de carbone encore contenus dans la protéine.

4. Utilisation des hydrolysats de protéines de blé accessible selon le procédé selon les revendications 1 à 3 en vue de la fabrication de dérivés de couleur claire, stables au stockage N-acylés, N-alkylés, estérifiés ainsi que N-acylés ou N-alkylés et en outre estérifiés.
